# EUROPÄISCHE PATENTSCHRIFT

(11) **EP 1 167 368 B1**
(45) Veröffentlichungstag und Bekanntmachung des Hinweises auf die Patenterteilung: **05.11.2003**
(21) Anmeldenummer: 01112846.9
(22) Anmeldetag: 31.05.2001
(51) Int. Cl.: C07D 495/04

(54) **Verfahren zur Herstellung von 3,4-Alkylendioxythiophen-2,5-dicarbonsäurederivaten**
Process for the preparation of 3,4-alkylenedioxythiophene-2,5-dicarboxylic acid derivatives
Procédé pour la préparation de dérivé de l'acide 3,4-alcoylèndioxythiophène-2,5-dicarboxylique

(30) Priorität: 13.06.2000 DE 10029075
(43) Veröffentlichungstag der Anmeldung: 02.01.2002
(73) Patentinhaber: Bayer Aktiengesellschaft, 51368 Leverkusen (DE)
(72) Erfinder: Jonas, Friedrich, Dr., 52066 Aachen (DE)

(56) Entgegenhaltungen:
- PEI Q. ET AL: "Electrochromic and highly stable poly(3,4-ethylenedioxythiophene) switches between opaque blue-black and transparent sky blue" POLYMER, Bd. 35, Nr. 7, 1994, XP001025972
- SANKARAN B ET AL: "HIGH-CONTRAST ELECTROCHROMIC POLYMERS FROM ALKYL-DERIVATIZED POLY(3,4-ETHYLENEDIOXYTHIOPHENES)" MACROMOLECULES, AMERICAN CHEMICAL SOCIETY. EASTON, US, Bd. 30, Nr. 9, 5. Mai 1997 (1997-05-05), Seiten 2582-2588, XP000688333 ISSN: 0024-9297
- LIMA A ET AL: "ELECTROPOLYMERIZATION OF 3,4-ETHYLENEDIOXYTHIOPHENE AND 3,4-ETHYLENEDIOXYTHIOPHENE METHANOL IN THE PRESENCE OF DODECYLBENZENESULFONATE" SYNTHETIC METALS, ELSEVIER SEQUOIA, LAUSANNE, CH, Bd. 93, 1998, Seiten 33-41, XP000995602 ISSN: 0379-6779

## Beschreibung

In der letzten Zeit haben organische leitfähige Polymere in der Technik mehr und mehr Verbreitung gefunden. Einsatzgebiete sind z.B. die Durchkontaktierung von Leiterplatten (EP-A-553671), Antistatikausrüstung fotografischer Filme (EP-A-440957) oder als Elektrode in Feststoffelektrolytkondensatoren (EP-A-340512). Besondere Bedeutung auf diesen Gebieten haben Poly-3,4-alkylendioxythiophene (EP-A-339340) erlangt, die sich durch hohe Stabilität und elektrische Leitfähigkeit auszeichnen. Die zu ihrer Herstellung erforderlichen monomeren 3,4-Alkylendioxythiophene können prinzipiell nach literaturbekannten Verfahren hergestellt werden. Eine Synthese ist z.B. in Gogte et al., Tetrahedron 23 (1967) 2437 beschrieben. Ausgehend von Thiodiessigsäuredieestern I und Oxalsäurediestem II werden entsprechend Formelschema 1 in Gegenwart von Alkalialkoholaten 3,4-Dihydroxythiophen-2,5-dicarbonsäureester III (Stufe 1) hergestellt. Diese werden dann mit Dihalogenalkanen alkyliert zu 3,4-Alkylendioxythiophen-2,5-dicarbonsäureestern IV (Stufe 2), die zu den 3,4-Alkylendioxythiophen-2,5- dicarbonsäuren V verseift (Stufe 3) und zu den monomeren 3,4-Alkylendioxythiophenen decarboxyliert werden. Die bisher nach dem Stand der Technik erfolgte Isolierung der einzelnen Stufen ist aufwendig und kostspielig.

Auch in Pei et al., Polymer 35 (1994) 1347 - 1351, Sankaran et al., Macromolecules 30 (1997) 2582 - 2588 und Chrevrot et al., Synthetic Metals 93 (1998) 33 - 41 wird die Herstellung von 3,4-Ethylendioxythiophen und dessen Derivaten beschrieben, wobei auch hier die aufwendige und kostspielige Isolierung der einzelnen Stufen erfolgt. Einzig Pei et al. verzichtet in einem Schritt auf die Isolation der 3,4-Ethylendioxythiophen-2,5-dicarbonsäureestern und verseift direkt zu den 3,4-Ethylendioxythiophen-2,5-dicarbonsäuren, jedoch führt diese Vorgehensweise zu erheblich verminderten Ausbeuten.

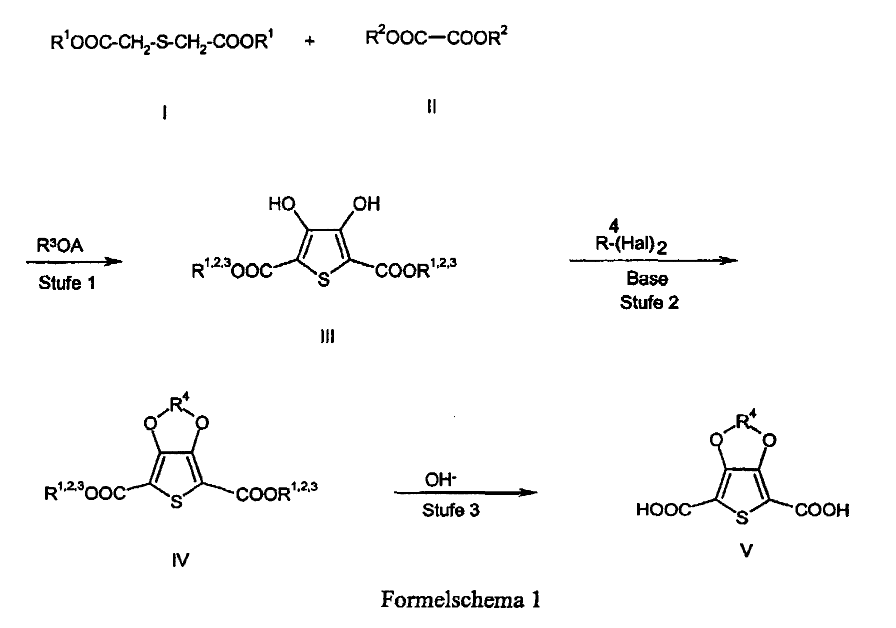

Im Formelschema 1 stehen:
- R¹, R², R³: gleich oder verschieden für einen linearen oder verzweigten, gegebenenfalls substituierten Alkylrest mit 1 bis 20 C-Atomen,
- R⁴: für einen linearen oder verzweigten, gegebenenfalls substituierten Alkylenrest mit 1 bis 20 C-Atomen,
- A: für Lithium, Natrium oder Kalium und
- Hal: für Fluor, Chlor, Brom oder Jod.

Überraschenderweise wurde jetzt gefunden, dass die Stufen 1 und 2 bzw. 2 und 3 bzw. 1 bis 3 ohne Isolierung der Zwischenprodukte durchgeführt werden können und die Zielprodukte dabei in hoher Reinheit und Ausbeute erhalten werden.

Gegenstand der Erfindung ist daher ein einfaches Verfahren zur Herstellung von 3,4-Alkylendioxythiophen-2,5-dicarbonsäuren bzw. deren Estern durch Zusammenfassung einzelner Synthesestufen ohne Isolierung der Zwischenstufen.

Das Verfahren wird im Folgenden näher beschrieben. Bei der Zusammenfassung der Stufen 1 und 2 wird folgendermaßen vorgegangen:

In Stufe 1 erfolgt die Kondensation der Thiodiessigsäureester mit Oxalsäureestern in Gegenwart von Alkalialkoholaten. Geeignete Alkalialkoholate sind die Alkoholate von Lithium, Natrium und Kalium, bevorzugt Natrium oder Kalium, die sich von linearen oder verzweigten aliphatischen Alkoholen ableiten. Bevorzugte Alkohole sind Methanol, Ethanol, Isopropanol, n und iso-Butanol, tert-Butanol.

Die Reaktion wird bevorzugt in Lösung durchgeführt. Geeignete Lösungsmittel sind niedere aliphatische Alkohole wie Methanol, Ethanol, Isopropanol, n und iso-Butanol, tert-Butanol. Bevorzugt wird der Alkohol verwendet, der auch in der Alkoholatkomponente enthalten ist. Thiodiessigsäureester und Oxalsäureester werden üblicherweise in äquimolaren Mengen eingesetzt. Bezogen auf 1 mol der Ester werden 2,0 bis 4,0 mol Alkoholat, bevorzugt 2,0 bis 3,0 mol Alkoholat, besonders bevorzugt 2,0 bis 2,5 mol Alkoholat verwendet.

Die Reaktion wird bei -10°C bis 200°C, bevorzugt 0°C bis 100°C, besonders bevorzugt 10°C bis 70°C durchgeführt.

Die Reaktionszeit liegt zwischen 10 Minuten und 24 Stunden, bevorzugt 1 Stunde und 8 Stunden.

Üblicherweise wird das Alkoholat vorgelegt und die Ester getrennt oder als Mischung unter Rühren zugetropft.

Nach Abschluss der Reaktion wird gegebenenfalls vorhandener Alkoholatüberschuss durch Zugabe von Säuren oder sauren Salzen wie Alkalihydrogensulfaten neutralisiert.

Für die Durchführung der 2. Stufe wird anschließend ein höher siedendes Lösungsmittel, bevorzugt mit einem Siedepunkt zwischen 100°C und 300°C zugesetzt. Geeignete Lösungsmittel sind z.B. lineare oder cyclische amidische Lösungsmittel wie N-Methylpyrrolidon, N,N-Dimethylformamid, N,N-Dimethylacetamid, aliphatische Sulfoxide oder Sulfone wie Dimethylsulfoxid oder Sulfolan. Die Lösungmittel können allein oder als Gemisch verwendet werden.

Nach Zugabe der höhersiedenden Lösungsmittel wird der Alkohol gegebenenfalls unter vermindertem Druck abdestilliert. Anschließend wird in einer bevorzugten Ausführungsform eine Base in einer Menge von 0,01 bis 0,5 mol bezogen auf 1 mol der eingesetzten Ester zugesetzt. Bevorzugte Basen sind Natrium und Kaliumcarbonat. Besonders bevorzugt ist Kaliumcarbonat.

Der Ringschluss zum 3,4-Alkylendioxythiophendicarbonsäureester erfolgt dann durch Umsetzung der Alkalisalze der 3,4-Dihydroxidicarbonsäureester mit Alkylierungsmitteln. Geeignete Alkylierungsmittel sind Dihalogenalkane. Bevorzugt werden Dichlor- oder Dibromalkane verwendet. Besonders bevorzugt sind lineare 1,2-Dihalogenalkane mit 2 bis 18 C-Atomen und 1,3-Dihalogenalkane mit 3 bis 18 C-Atomen (Halogen ist gleich oder verschieden Fluor, Chlor, Brom, Jod). Besonders bevorzugt ist 1,2-Dichlorethan und 1,2-Dichlor-hexadecan.

Die Umsetzung erfolgt bei Temperaturen von 50 bis 200°C, bevorzugt bei 100 bis 150°C, gegebenenfalls unter Druck. Die Reaktionsdauer liegt zwischen 1 und 24 Stunden.

Nach vollständiger Reaktion wird der 3,4-Alkylendioxythiophendicarbonsäureester durch Abdestillieren des Lösungsmittels gegebenenfalls unter vermindertem Druck und/oder Fällung mit Wasser isoliert. Das Rohprodukt kann anschließend getrocknet werden oder direkt in feuchtem Zustand zur freien 3,4-Alkylendioxythiophendicarbonsäure verseift werden.

In einer besonderen Ausführungsform der Erfindung wird unter Einbeziehung der Stufe 3 der 3,4-Ethylen-dioxthiophencarbonsäureester nicht isoliert. Nach vollständiger Alkylierung wird der größte Teil des Lösungsmittels gegebenenfalls unter vermindertem Druck abdestilliert und anschließend der 3,4-Ethylendioxthiophencarbonsäureester direkt mit Basen verseift.

Vorzugsweise werden für die Verseifung Alkalihydroxide eingesetzt, die als Lösung in Wasser und/oder im Gemisch mit wassermischbaren aliphatischen Alkoholen zum Einsatz kommen. Geeignete Alkohole sind z.B. Methanol, Ethanol und Isopropanol. Die Verseifung kann bei Raumtemperatur oder Temperaturen oberhalb Raumtemperatur erfolgen. Bewährt hat sich die Verseifung bei Rückflusstemperatur des Wassers bzw. des Wasser/Alkoholgemisches. Nach vollständiger Verseifung wird durch Zugabe von Mineralsäuren die freie 3,4-Ethylendioxythiophen-dicarbonsäure freigesetzt und ausgefällt. Das Produkt wird anschließend durch Absaugen isoliert und getrocknet.

In einer weiteren Ausführungsform werden die Stufen 2 und 3 zusammengefasst. Zur Durchführung der 2. Stufe wird der getrennt hergestellte 3,4-Dihydroxythiophen-2,5-dicarbonsäureester in den oben beschriebenen Lösungsmitteln vorgelegt. Als Base werden bezogen auf 1 mol 3,4-Dihydroxythiophen-2,5-dicarbonsäureester 1,0 bis 1,5 mol, bevorzugt 1,1 bis 1,4 mol Alkalicarbonat zugegeben. Bevorzugt wird Kaliumcarbonat verwendet.

Die weitere Umsetzung zum 3,4-Alkylendioxythiophendicarbonsäureester und Verseifung zur 3,4-Alkylendioxythiophendicarbonsäure erfolgt wie weiter oben bei der Zusammenfassung der Stufen 1 bis 3 beschrieben.

### Beispiel 1

### 3,4-Ethylendioxythiophen-2,5-dicarbonsäureester-Gemisch aus Dimethyl-, Methylethyl- und Diethylester

### (Zusammenfassung der 1. und 2. Stufe)

In einer 6 l-Planschliffapparatur, versehen mit Gitterrührer, Rückflusskühler, Thermometer und Tropftrichter, werden 848 g 32 %ige Kalium-methylatlösung (3,88 mol) und 200 g Methanol vorgelegt und anschließend bei 0 bis 20°C eine Mischung aus 377,3 g Thiodiessigsäurediethylester 95 %ig (1,74 mol) und 254 g Oxalsäure-diethylester (1,74 mol) innerhalb von 60 min zugetropft. Anschließend wird 2 Stunden bei Raumtemperatur, 1 Stunde bei 40°C und 3 Stunden bei Rückfluss gerührt. Die abgekühlte Suspension wird mit 47,6 g Kaliumhydrogensulfat (0,35 mol) versetzt und 10 Minuten gerührt. Unter Rühren werden 1700 g N,N-Dimethylformamid (DMF) und 200 g Dimethylsulfoxid (DMSO) zugegeben. Das Methanol wird im Wasserstrahlvakuum über eine 10 cm Kolonne abdestilliert bis die Kopftemperatur 40°C bei 100 mbar erreicht. Nach Zugabe von 27,6 g Kaliumcarbonat (0,2 mol) werden bei 80°C 168,4 g 1,2-Dichlorethan (1,7 mol) in 2 Stunden zugetropft. Die Suspension wird 2 Stunden bei 100°C und 10 Stunden bei 125°C gerührt. Anschließend werden in 1 Stunde 48 g 1,2-Dichlorethan (0,48 mol) bei 125°C zugetropft. Die Reaktion wird in 8 Stunden bei 130 bis 135°C zu Ende geführt. Nach Abkühlen auf ca 70°C werden ca 1030 g DMF/1,2-Dichlorethangemisch im Wasserstrahlvakuum abdestilliert. Nach dem Abkühlen wird der Reaktionsansatz in 4 l Eiswasser eingerührt. Nach einer Nachrührzeit von 30 min wird das Produkt abgesaugt.

Das Rohprodukt wird einmal in ca. 1,5 l Wasser unter Rühren aufgeschlämmt und abgesaugt.

Ausbeute (getrocknet): 417 g = 93 % d. Th.(Restsalzgehalt nicht berücksichtigt)

### Beispiel 2

### 3,4-Ethylendioxythiophen-2,5-dicarbonsäure

### (Zusammenfassung der 2. und 3. Stufe)

In einem 4 l-Planschliffgefäß versehen mit Rückflusskühler mit Gasableitung, Thermometer, Rührer und beheizbarem Tropftrichter werden 980 g DMF, 78 g DMSO und 296,2 g Kaliumcarbonat (2,15 mol) vorgelegt. Bei 80 bis 90°C wird eine Lösung aus 409 g des Ester-Gemisches aus Beispiel 1 (1,76 mol) und 212,4 g 1,2-Dichlorethan (2,14 mol) in 980 g DMF (bei 50°C gelöst) in 2 Stunden zugetropft (CO₂-Entwicklung). Die Suspension wird 2 Stunden bei 100°C und 10 Stunden bei 125°C gerührt. Anschließend werden in 1 Stunde 49,5 g 1,2-Dichlorethan (0,5 mol) bei 125°C zugetropft. Die Reaktion wird in 8 Stunden bei 130 bis 135°C zu Ende geführt. Nach Abkühlen auf ca 70°C werden ca. 1700 g DMF/1,2-Dichlorethangemisch im Wasserstrahlvakuum abdestilliert. Bei 50 bis 60°C werden in 2 Stunden 1107 g 15% ige Natronlauge (4,15 mol) unter Rühren zugetropft. Anschließend wird 14 Stunden bei 60°C weitergerührt. Nach dem Abkühlen werden unter Eiskühlung 2646 g 10 %ige Schwefelsäure (2,7 mol) zugetropft, bis ein pH-Wert von 1 erreicht ist. Nach einer Nachrührzeit von 1 Stunde wird das Produkt abgesaugt Das Rohprodukt wird in 3 1 Wasser aufgeschlämmt und abgesaugt.

Das Produkt wird 24 Stunden bei 80°C und anschließend im Vakuumtrockenschrank (80°C) bis zur Gewichtskonstanz getrocknet.

Ausbeute: 369,1 g = 91 % d. Th. (Restsalzgehalt nicht berücksichtigt)

### Beispiel 3

### 3,4-Ethylendioxythiophen-2,5-dicarbonsäure

### (Zusammenfassung der 1. bis 3. Stufe)

In einer 6 l-Planschliffapparatur, versehen mit Gitterrührer, Rückflusskühler, Thermometer und Tropftrichter, werden 1181,2 g 32 %ige Kalium-methylatlösung (5,4 mol) und 300 g Methanol vorgelegt und anschließend bei 0 bis 20°C eine Mischung aus 552,9 g Thiodiessigsäurediethylester 95 %ig (2,55 mol) und 372,3 Oxalsäure-diethylester (2,55 mol) innerhalb von 60 min zugetropft. Anschließend wird 2 Stunden bei Raumtemperatur, 1 Stunde bei 40°C und 3 Stunden bei Rückfluss gerührt. Die abgekühlte Suspension wird mit 42 g Natriumhydrogensulfat (0,35 mol) versetzt und 10 Minuten gerührt. Unter Rühren werden 2500 g DMF und 250 g DMSO zugegeben. Das Methanol wird im Wasserstrahlvakuum über eine 10 cm Kolonne abdestilliert bis die Kopftemperatur 40°C bei 100 mbar erreicht. Nach Zugabe von 37,5 g Kaliumcarbonat (0,27 mol) werden bei 80°C 233,5 g 1,2-Dichlorethan (2,36 mol) in 2 Stunden zugetropft. Die Suspension wird 2 Stunden bei 100°C und 10 Stunden bei 125°C gerührt. Anschließend werden in 1 Stunde 155 g 1,2-Dichlorethan (1,56 mol) bei 125°C zugetropft. Die Reaktion wird in 8 Stunden bei 130 bis 135°C zu Ende geführt. Nach Abkühlen auf ca. 70°C werden ca 1800 g DMF/1,2-Dichlorethangemisch im Wasserstrahlvakuum abdestilliert. Bei 50 bis 60°C werden in 2 Stunden 3500 g 15% ige Natronlauge (13,1 mol) unter Rühren zugetropft. Anschließend wird 14 Stunden bei 60°C weitergerührt. Nach dem Abkühlen werden unter Eiskühlung 7644 g 10 %ige Schwefelsäure (7,8 mol) zugetropft, bis ein pH-Wert von 1 erreicht ist. Nach einer Nachrührzeit von 1 Stunde wird das Produkt abgesaugt. Das Rohprodukt wird in 3 1 Wasser aufgeschlämmt und abgesaugt.

Das Produkt wird 24 Stunden bei 80°C und anschließend im Vakuumtrockenschrank (80°C) bis zur Gewichtskonstanz getrocknet.

Ausbeute: 453,1 g = 77 % d. Th. (Restsalzgehalt nicht berücksichtigt)

## Patentansprüche

1. Verfahren zur Herstellung von 3,4-Alkylendioxythiophen-2,5-dicarbonsäurederivaten der allgemeinen Formeln IV und V, 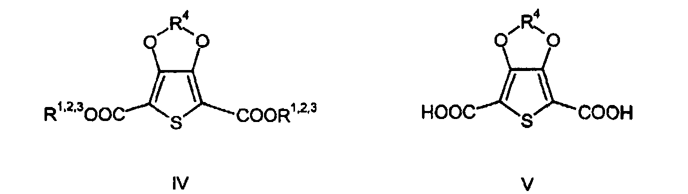 **dadurch gekennzeichnet, dass** mindestens 2 Stufen gemäß dem Formelschema 1, in dem R¹, R², R³, gleich oder verschieden für einen linearen oder verzweigten, gegebenenfalls substituierten Alkylrest mit 1 bis 20 C-Atomen, R⁴ für einen linearen oder verzweigten, gegebenenfalls substituierten Alkylenrest mit 1 bis 20 C-Atomen, A für Lithium, Natrium oder Kalium und Hal für Fluor, Chlor, Brom oder Jod stehen, ohne Isolierung der Zwischenprodukte durchgeführt werden und die Umsetzung des 3,4-Dihydroxydicarbonsäureesters oder dessen Alkalisalzes mit dem Alkylierungsmittel R⁴-(Hal)₂ in Gegenwart der Base bei Temperaturen von 100 bis 150°C erfolgt.

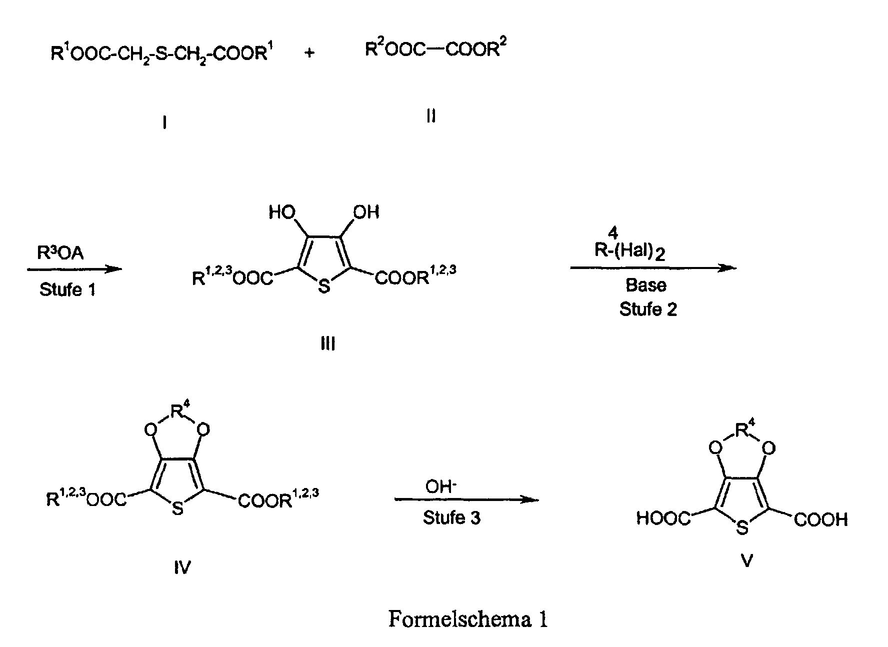

2. Verfahren nach Anspruch 1, **dadurch gekennzeichnet, dass** in Stufe 2 das Dikaliumsalz des 3,4-Dihydroxythiophen-2,5-dicarbonsäureesters eingesetzt wird.

3. Verfahren nach Anspruch 1 oder 2, **dadurch gekennzeichnet, dass** die Stufe 2 in Gegenwart eines Überschusses an Base durchgeführt wird.

4. Verfahren nach Anspruch 3, **dadurch gekennzeichnet**, das als Base Kaliumcarbonat verwendet wird.

5. Verfahren nach Anspruch 1, **dadurch gekennzeichnet, dass** 3,4-Ethylendioxythiophen-2,5-dicarbonsäure hergestellt wird.

6. Verfahren nach wenigstens einem der Ansprüche 1 bis 5, **dadurch gekennzeichnet, dass** für die Durchführung der Stufe 2 ein Lösungsmittel mit einem Siedepunkt zwischen 100 und 300°C zugesetzt wird.

7. Verfahren nach Anspruch 6, **dadurch gekennzeichnet, dass** als Lösungsmittel lineare oder cyclische amidische Lösungsmittel, aliphatische Sulfoxide oder Sulfone allein oder im Gemisch verwendet werden.

## Claims

1. Process for the preparation of 3,4-alkylenedioxythiophene-2,5-dicarboxylic acid derivatives of the general formulae IV and V 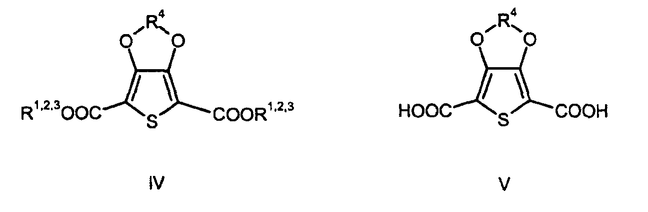 **characterized in that** at least 2 steps in accordance with formula scheme 1, in which R¹, R² and R³ are identical or different and are a linear or branched, optionally substituted alkyl radical having 1 to 20 carbon atoms, R⁴ is a linear or branched, optionally substituted alkylene radical having 1 to 20 carbon atoms, A is lithium, sodium or potassium, and Hal is fluorine, chlorine, bromine or iodine, are carried out without isolation of the intermediates, and the reaction of the 3,4-dihydroxydicarboxylic acid ester or its alkali metal salt with the alkylating agent R⁴-(Hal)₂ is carried out in the presence of a base at from 100 to 150°C.

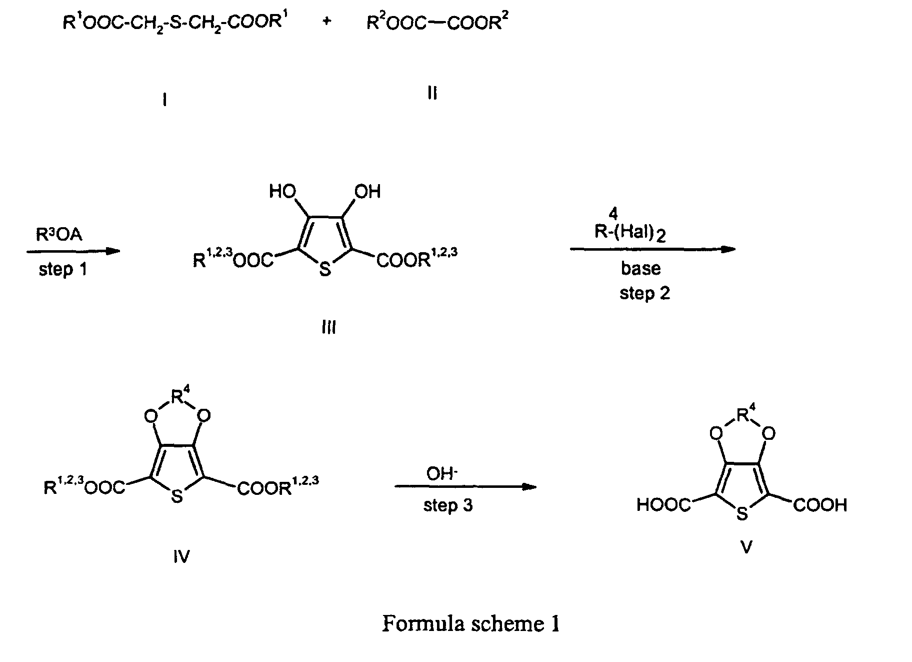

2. Process according to Claim 1, **characterized in that** the dipotassium salt of the 3,4-dihydroxythiophene-2,5-dicarboxylic acid ester is employed in step 2.

3. Process according to Claim 1 or 2, **characterized in that** step 2 is carried out in the presence of an excess of base.

4. Process according to Claim 3, **characterized in that** the base used is potassium carbonate.

5. Process according to Claim 1, **characterized in that** 3,4-ethylenedioxythiophene-2,5-dicarboxylic acid is prepared.

6. Process according to at least one of Claims 1 to 5, **characterized in that** a solvent having a boiling point of from 100 to 300°C is added for carrying out step 2.

7. Process according to Claim 6, **characterized in that** the solvents used are linear or cyclic amidic solvents, aliphatic sulphoxides or sulphones, alone or as mixtures.

## Revendications

1. Procédé pour la préparation de dérivés d'acides 3,4-alkylènedioxythiophène-2,5-dicarboxyliques de formules générales IV et V, 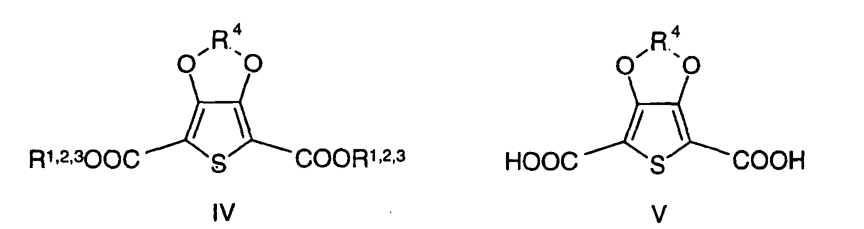 **caractérisé en ce que** l'on exécute au moins deux stades du schéma de réaction 1 ci-après dans lequel R¹, R², R³, ayant des significations identiques ou différentes, représentent chacun un groupe alkyle linéaire ou ramifié éventuellement substitué en C₁-C₂₀, R⁴ représente un groupe alkylène linéaire ou ramifié éventuellement substitué en C₁-C₂₀, A représente le lithium, le sodium ou le potassium et Hal représente le fluor, le chlore, le brome ou l'iode, sans isoler les produits intermédiaires, et on exécute la réaction de l'ester 3,4-dihydroxydicarboxylique ou de son sel alcalin avec l'agent alkylant R⁴-(Hal)² en présence de bases à des températures de 100 à 150°C :

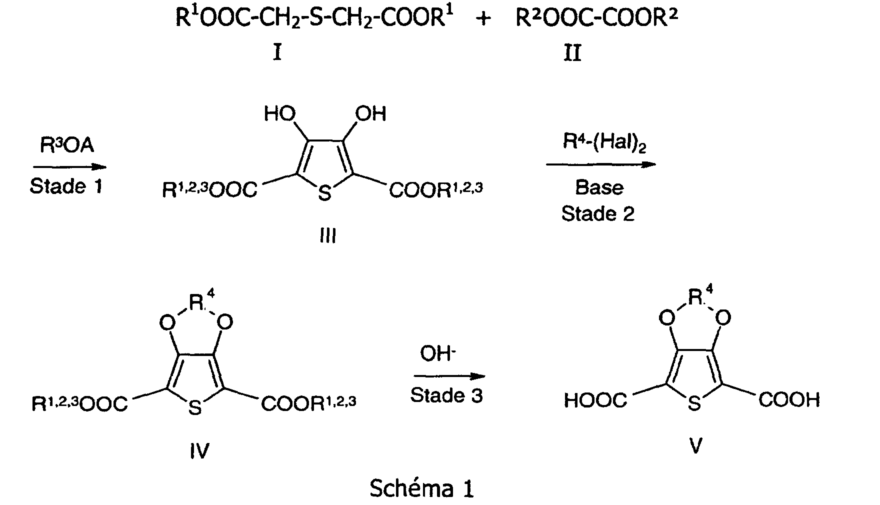

2. Procédé selon la revendication 1, **caractérisé en ce que**, au stade 2, on met en oeuvre le sel dipotassique de l'ester 3,4-dihydroxythiophène-2,5-dicarboxylique.

3. Procédé selon la revendication 1 ou 2, **caractérisé en ce que**, au stade 2, on opère en présence d'un excès d'une base.

4. Procédé selon la revendication 3, **caractérisé en ce que** la base utilisée est le carbonate de potassium.

5. Procédé selon la revendication 1, **caractérisé en ce que** l'on prépare l'acide 3,4-éthylènedioxythiophène-2,5-dicarboxylique.

6. Procédé selon au moins une des revendications 1 à 5, **caractérisé en ce que** pour l'exécution du stade 2, on ajoute un solvant dont le point d'ébullition va de 100 à 300°C.

7. Procédé selon la revendication 6, **caractérisé en ce que** le solvant utilisé consiste en un amide linéaire ou cyclique, un sulfoxyde ou une sulfone aliphatiques, isolément ou en mélange entre eux.
